# NEW EUROPEAN PATENT SPECIFICATION

(11) **EP 0 167 168 B2**
(45) Date of publication and mention of the opposition decision: **02.07.1997**
(45) Mention of the grant of the patent: 15.11.1989
(21) Application number: 85108303.0
(22) Date of filing: 04.07.1985
(51) Int. Cl.: G03C 7/32, C07D 249/18, C07D 257/04, C07D 403/06

(54) **Silver halide photographic material**
Photographisches Silberhalogenidmaterial
Matériau photographique à l'halogénure d'argent

(30) Priority: 04.07.1984 JP 138808/84; 04.04.1985 JP 71768/84
(43) Date of publication of application: 08.01.1986
(73) Proprietor: FUJI PHOTO FILM CO., LTD., Kanagawa 250-01 (JP)
(72) Inventor: Adachi, Keiichi, Minami Ashigara-shi Kanagawa (JP); Sato, Shingo, Minami Ashigara-shi Kanagawa (JP)
(74) Representative: Grünecker, Kinkeldey, Stockmair & Schwanhäusser Anwaltssozietät

(56) References cited:
- DE-A- 2 417 914
- DE-A- 2 952 280
- DE-A- 3 209 486
- DE-A- 3 506 805
- FR-A- 2 239 706
- US-A- 3 379 529
- US-A- 3 620 746
- US-A- 4 144 071
- US-A- 4 345 024

## Description

The present invention relates to a sliver halide photographic material containing a group which may release a photographically useful group through an oxidation step during photographic processing.

Numerous compounds are known which release photographically useful groups in accordance with the image density during development. Hydroquinone derivatives (e.g., DIR hydroquinone) are known as compounds which release development restrainers in correspondence to image density during development. Exemplary compounds known as DIR hydroquinones include those described in U.S. Patents 3,379,529 and 3,620,746 and Jaoanese Patent Application (OPI) No. 129,536/74. (The term "OPI" as used herein refers to a "published unexamined Jaoanese patent application.")

As is known from the above-cited references, DIR hydroquinone is used to obtain the so-called DIR effects, such as improvements in reproducibility of tone of the image, graininess of the image, sharpness of the image and color reproducibility. Conventional DIR hydroquinones snow a certain degree of these effects. but further imorovement has been required.

In particular, known DIR hydroquinones nave the following disadvantage:

As the restrainer released during development becomes diffused from the sensitive material into the deveiooing solution, it is accumuatea in the solution. As a result, the solution shows restraining effects. If a large quantity of the sensitive material is treatea on a continuous basis, as in the method commonly emoioyed on a commercial scale, it is difficult to alwavs obtain a constant color tone. Thus, the contamination of the development solution with the restrainer release by DIR hydroquinone is a serious problem.

In order to solve this problem, various measures have been taken as a matter of convenience. but they all have some disadvantages and no fundamental solution has not yet been known. For example, the following methods are known:
(1) Restriction of the amount of DIR hydroquinone used for the development;
(2) Frequent change of the developer solution for a new supply; and
(3) Incorporation in the sensitive material of a microcrystal emulsion layer free from anv substantial photographic sensitivity as a scavenger for the restrainer released from the sensitive layer.

However, these methods all have disadvantages such as the limitation of photographic improvement and a large increase in cost.

It is the object of the present invention to provide a photographic material which shows excellent performance in coior reproduction ana gives excellent sharpness to the image.

Said object is achieved by a silver halide photographic material according to claim 1.

The compound having a group which after being released from the compound by redox reaction is converted into a compound having a development restraining ability and which is further converted in the developing solution into a compound which has no substantial development ability or which shows a marked decrease in such ability is represented by the general formula (I): 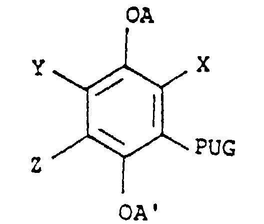 wherein A and A' are each hydrogen or a group which can be hydrolyzed by alkali, such as acyl or alkoxycarbonyl group; X, Y and Z are each hydrogen, a halogen atom (e.g. chlorine, bromide and iodine), a substituted or unsubstituted alkyl group (preferably having 1 to 20 carbon atoms, such as methyl, ethyl, propyl, aryl, octyl, dodecyl, cyclohexyl, hydroxyethyl, sulfonylethyl and carboxypropyl groups), a substituted or unsubstituted alkoxy group (preferably having 1 to 20 carbon atoms, such as methoxy, ethoxy, butyloxy, octyloxy, hexadecyloxy and methoxyethoxy groups), a substituted or unsubstituted aryl group (preferably having 1 to 20 carbon atoms, such as dodecylphenyl, tolyl, p-methoxyphenyl and p-chlorophenyl groups) or an alkylthio group (preferably having 6 to 20 carbon atoms, such as methylthio, butylthio, octylthio and hexadecylthio groups), X may additionally be an electron-withdrawing group selected from 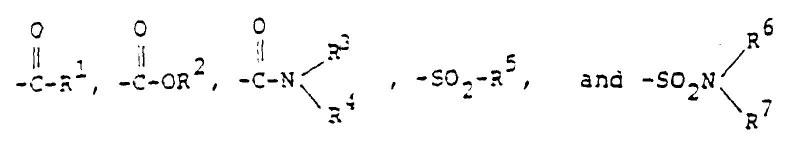 wherein R¹ to R⁷ are each a substituted or unsubstituted alkyl group (preferably having 1 to 30 carbon carbon atoms, such as methyl, ethyl propyl, aryl, butyl, octyl, dodecyl, adamantyl, cyclohexyl, methoxyethyl, hydroxyethyl, sulfoethyl and carboxypropyl groups), a substituted or unsubstituted aryl group (preferably having 6 to 30 carbon atoms, such as dodecylphenyl, tolyl, p-methoxyphenyl, phenyl, p-chlorophenyl and m-nitrophenyl groups) and a substituted or unsubstituted aralkyl group (preferably having 7 to 30 carbon atoms, such as benzyl and phenethyl groups); or Y and Z may form a 5- or 6-membered ring through a methylene chain.

In formula (I), PUG is a group released by a reaction with a light-exposed silver halide and/or the oxidized product of the developing agent and is bonded to the benzene nucleus via a sulfur atom or a nitrogen atom,
wherein said PUG is selected from the group consisting of formulae (P-1) and (P-2) 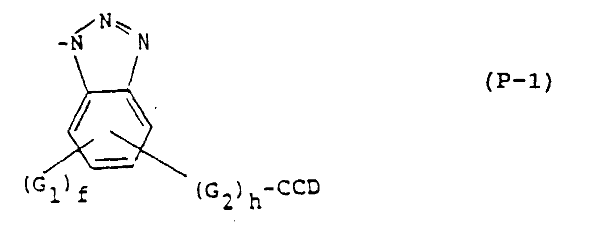 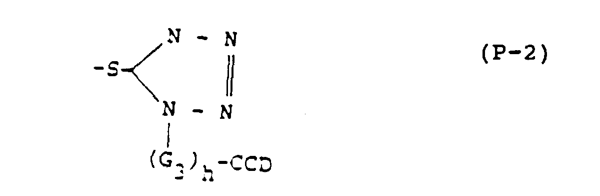
wherein G₁ is hydrogen, a halogen atom, an alkyl group (e.g. methyl and ethyl groups), an acylamino group (e.g. benzamido and hexanamido groups), an alkoxy group (e.g. methoxy and benzyloxy groups); a sulfonamido group (e.g. methanesulfonamido and benzenesulfonamido groups) an aryl group (e.g. phenyl and 4-chlorophenyl groups), an alkylthio group (e.g. methylthio and butylthio groups), an alkylamino group (e.g. a cyclohexylamino group), an anilino group (e.g. anilino and 4-methoxycarbonylanilino groups), an amino group, an alkoxycarbonyl group (e.g. methoxycarbonyl and butoxycarbonyl groups), an acyloxy group (e.g. acetyl, butanoyl and benzoyl groups), a nitro group, a cyano group, a sulfonyl group (e.g. butanesulfonyl and benzenesulfonyl groups), an aryloxy group (e.g. phenoxy and naphthyloxy groups), a hydroxy group, a thioamido group (e.g. butanethioamido and benzenethiocarbonamido groups), a carbamoyl group (e.g. carbamoyl and N-arylcarbamoyl groups), a sulfamoyl group (sulfamoyl and N-arylsulfamoyl groups), a carboxyl group, a ureido group (e.g. ureido and N-ethylureido groups) or an aryloxycarbonyl group (e.g. phenoxycarbonyl and 4-methoxycarbonyl groups);
wherein G₂ is any one of the substituents listed for G₁ which may form a divalent group;
wherein G₃ is a substituted or unsubstituted alkylene group or substituted or unsubstituted arylene group and may be interrupted by a divalent ether, ester, thioether, amido, ureido, imido, sulfon or sulfonamido bond or carbonyl group, wherein said bonding groups and alkylene and arylene groups may be linked together to form a divalent group;
wherein f is an integer of 1 or 2, and h is 0 or 1. When f is 2, the two G₁'s may or may not be identical.

In formulae (P-1) and (P-2), when G₁, G₂ or G₃ contains an alkyl group portion, the alkyl group has 1 to 22, and preferably 1 to 10 carbon atoms and may be substituted or unsubstituted, straight-chain or branched, a chain or a ring, or saturated or unsaturated. When G₁, G₂ or G₃ contains an aryl group portion, the aryl group has 6 to 10 carbon atoms and is preferably a substituted or unsubstituted phenyl group.

The group represented by CCD is shown by the following formula (D-1):

-COOR⁸ (D-1)

wherein R⁸ is a substituted or unsubstituted alkyl group (preferably having 1 to 10 carbon atoms, such as methyl, ethyl, 2,3-dichloropropyl, 2,2,3,3-tetrafluoropropyl, butoxycarbonyl, methylcyclohexylaminocarbonylmethyl, methoxyethyl and propargyl groups), a substituted or unsubstituted aryl group (preferably having 6 to 10 carbon atoms, such as phenyl, 3,4-methyleneoxyphenyl, p-methoxyphenyl, p-cyanophenyl and m-nitrophenyl groups), or a substituted or unsubstituted aralkyl group (preferably having 7 to 12 carbon atoms, such as benzyl and p-nitrobenzyl groups).

Exemplary compounds used in the present invention are the following.

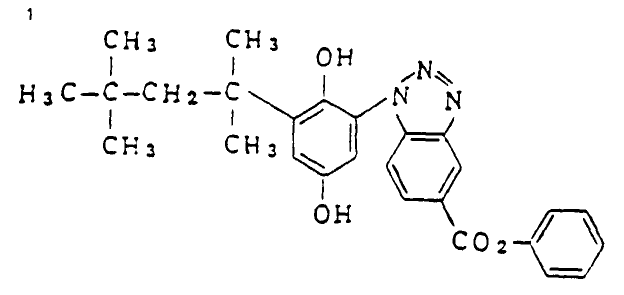

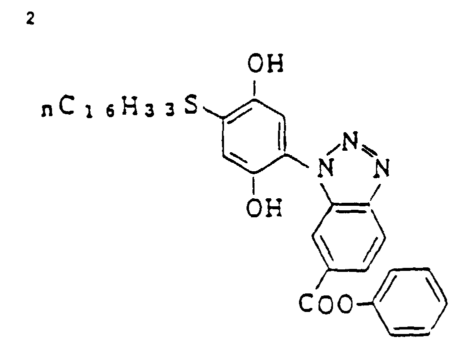

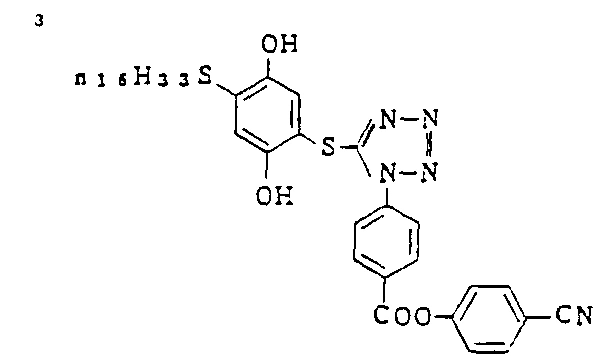

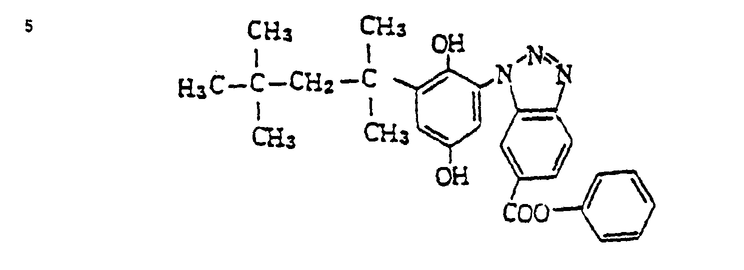

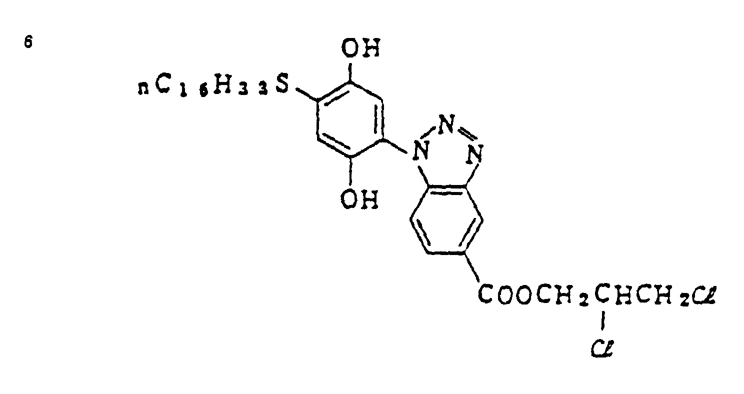

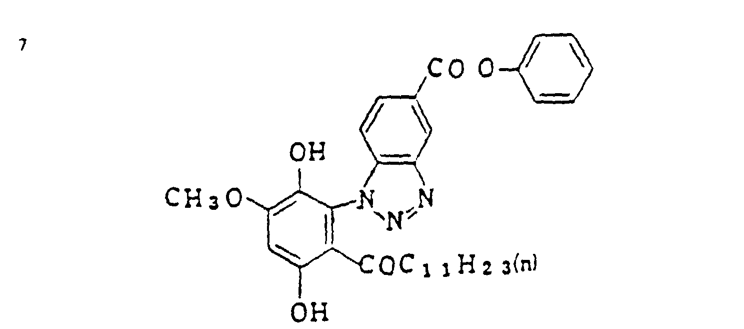

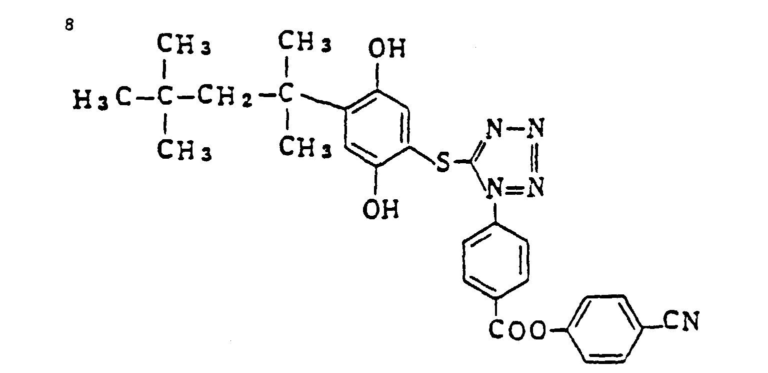

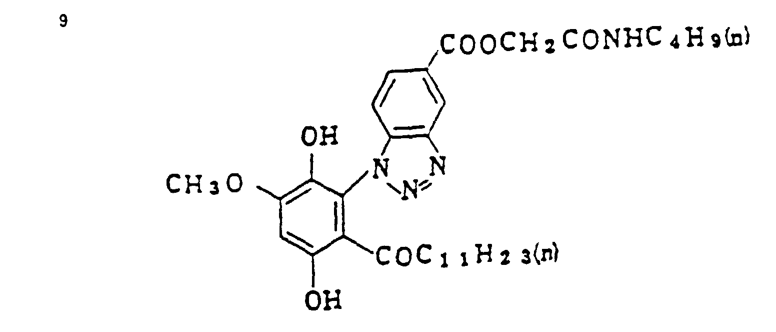

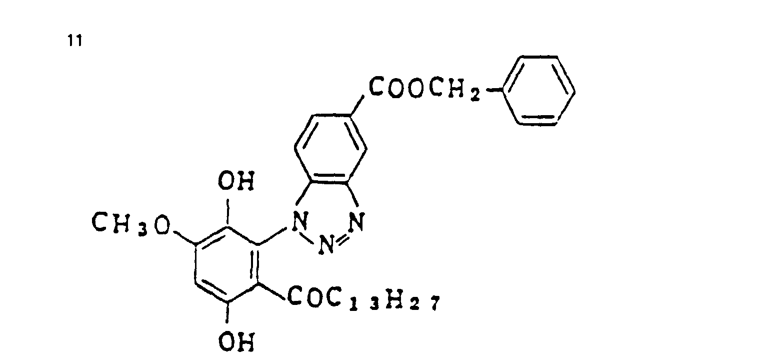

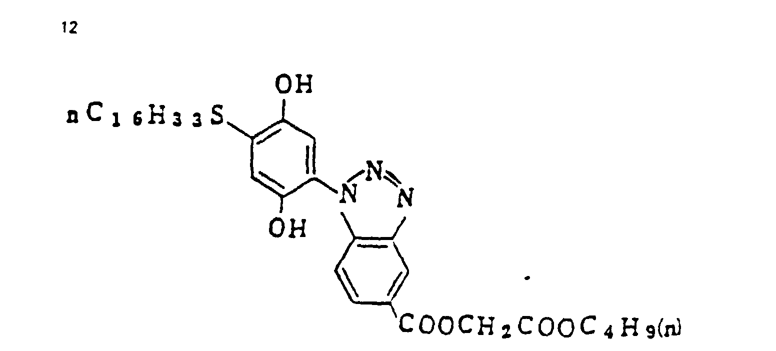

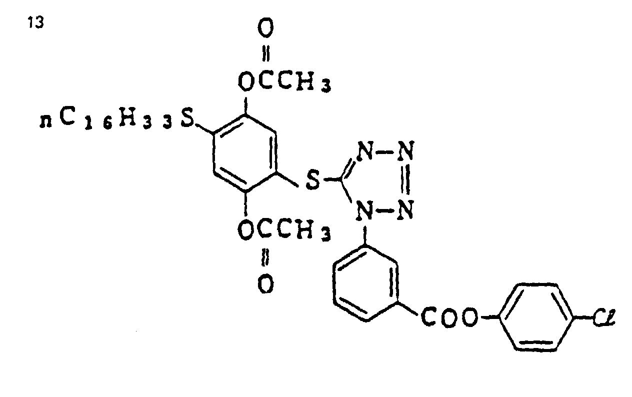

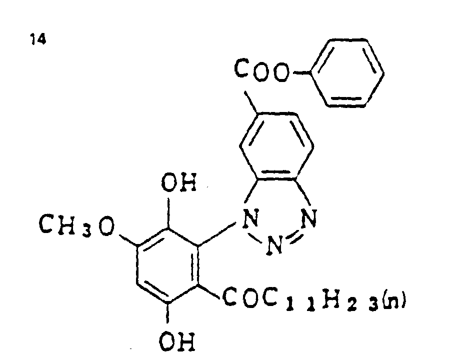

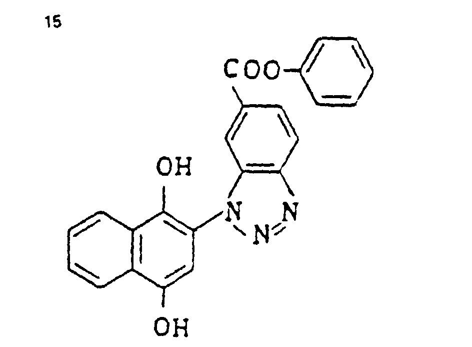

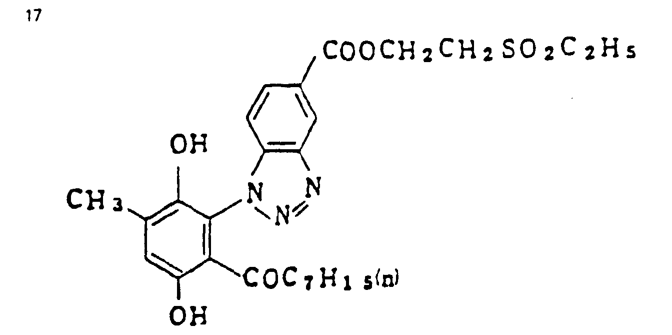

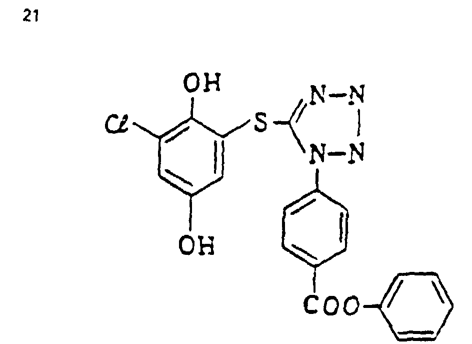

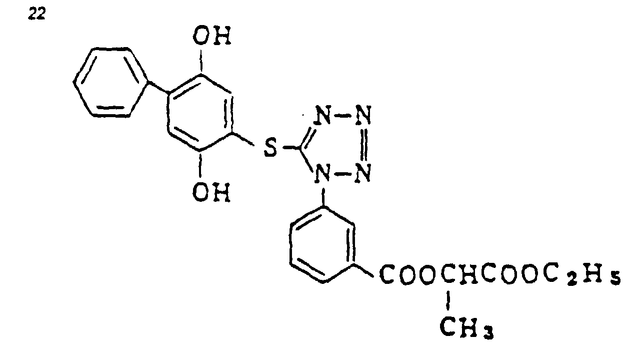

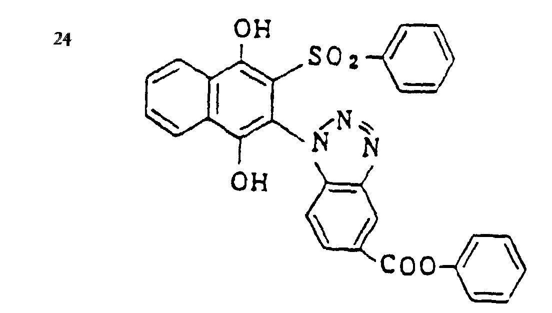

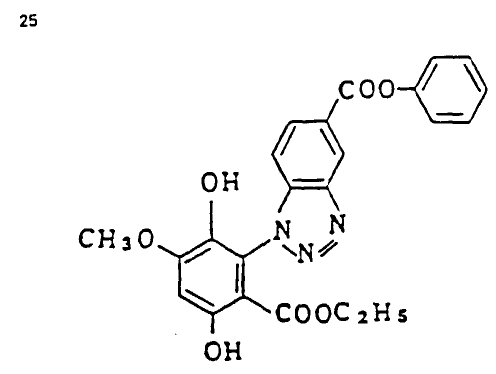

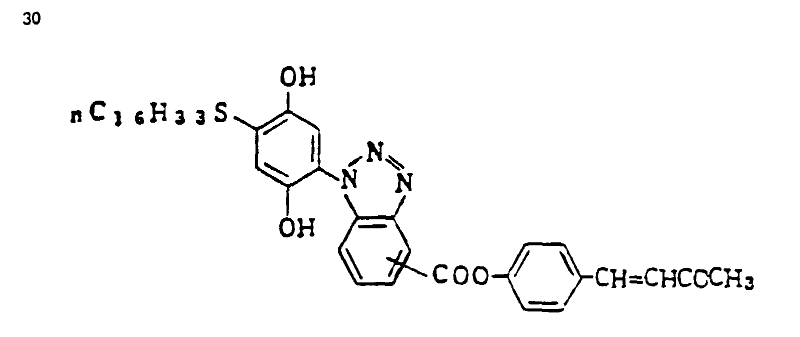

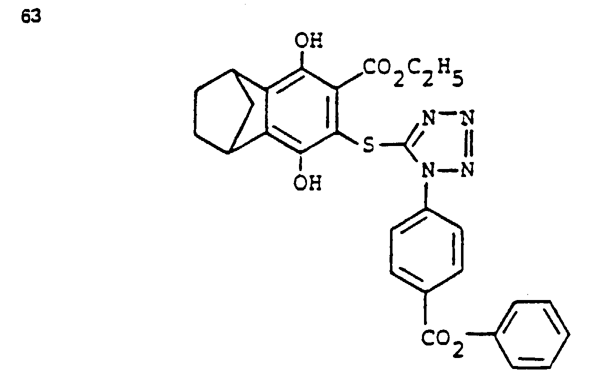

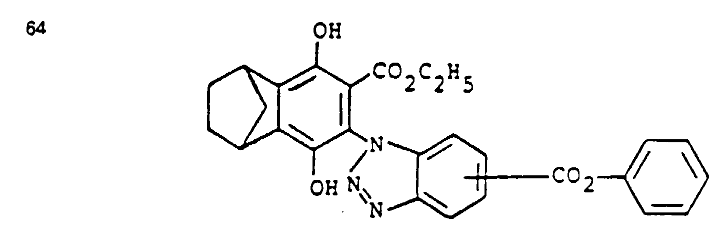

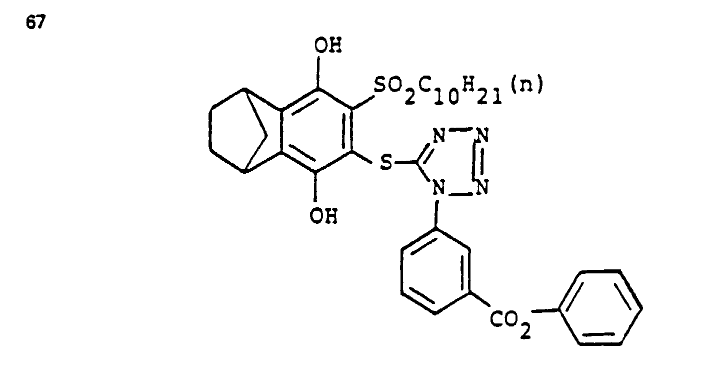

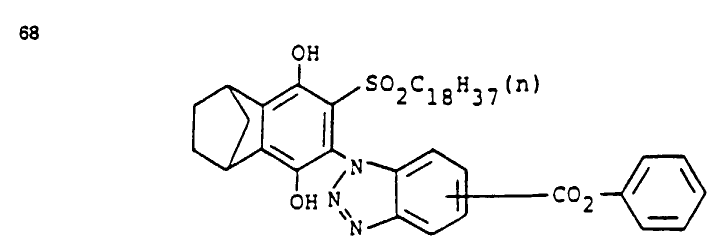

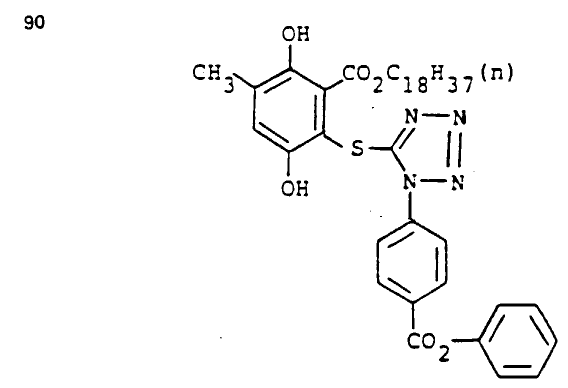

The compound shown by the formula (I) may generally be synthesized by the following two methods:
1) By reacting a benzoquinone derivative with a mercaptoazole or benzotriazole derivative in chloroform, 1,2-dichloroethane or carbon tetrachloride and in the presence of an acid catalyst such as p-toluenesulfonic acid, benzenesulfonic acid, trifluoromethanesulfonic acid or methanesulfonic acid at a 5 temperature ranging from room temperature to 100°C.
2) By reacting a chlorine-, bromine- or iodine-substituted benzoquinone derivative with a photographically useful compound in a non-protonic polar solvent such as acetone, tetrahydrofuran and dimethylformamide and in the presence of a base such as potassium carbonate, sodium bicarbonate and sodium hydroxide at a temperature ranging from -20°C to 100°C to obtain a quinone form and reducing it with a reducing agent such as diethylhydroxylamine or sodium hydrosulfite. (See Research Disclosure 18227 (1979), and Liebigs Ann. Chem., 764, 131 (1972).)

Specific examples of synthesizing the compounds used in the present invention are shown below. The compounds used in the present invention can be easily synthesized by these methods.

### Synthesis Example 1 (synthesis of Compound No. 1)

After mixing tertiary octylbenzoquinone (20g) and 5-phenoxycarbonylbenzotriazole (22 g) in acetonitrile (200 ml), a catalytic amount of p-toluenesulfonic acid was further added. The resulting mixture was refluxed under a nitrogen stream for about 8 h. The solvent was concentrated and the residue was purified through the silica gel chromatography. A 4:1 mixture of hexane and ethyl acetate was used as the solvent. The first fraction was concentrated and the residue was crystallized from acetonitrile to obtain 4.3 g of Compound No. 1 (m.p.: 205-207°C).

### Synthesis Example 2 (synthesis of Compound No. 3)

p-benzoquinone (20 g and 1-[4-(4-cyanophenoxycarbonyl)phenyll-5-mercaptotetrazole (40 g) were mixed in acetonitrile (300 ml) and reacted at room temperature. The resultant crystals were filtered off and the obtained crystals were oxidized with manganese dioxide in acetone. The excess oxidizing agent was filtered off, the filtrate was concentrated, and the residue was crystallized from an aqueous acetone to obtain 20 g of a quinone form (m.p.: 173-174°C). 20 g of this obtained quinone form and (nlhexadecylmercaptane (11 g) were mixed in acetonitrile (200 ml), and a catalytic amount of p-toluenesulfonic acid was further added and the resulting mixture was reacted at room temperature for 5 h. The precipitated crystals were filtered off, the filtrate was concentrated and the residue was crystallized from acetonitrile to obtain 8 g of Compound No. 3. (m.p.: 98-99°C).

### Synthesis Example 3 (synthesis of Compound No. 5)

A mixture similar to that of Example 1 was prepared and p-toluenesulfonic acid was added. The resultant mixture was refluxed under a nitrogen stream for 3 h. After the acetonitrile was concentrated, the residue was purified through silica gel chromatography. A 3:1 mixture of hexane and ethyl acetate was used as the solvent and the first small fraction was removed. The second fraction was concentrated and crystallized from acetonitrile to obtain 3.5 g of Compound No. 5. (m.p.: 207-209°C).

### Synthesis Example 4 (synthesis of Compounds Nos. 7 and 14)

5-phenoxycabonyl benzotriazole (20 g) was added to an acetonitrile solution of 5-methoxy-2-undecanoylbenzoquinone (15 g), and the resultant solution was stirred at room temperature for 2 h. The resulting reaction mixture was dropwise added to an excess Na₂S₂O₅ solution and stirred for 1 h. The obtained crystals were filtered and washed with water and crystallized from acetonitrile to obtain 3 g of the first compound (compound No. 7) (m.p. 162-164°C). Then the filtrate was left to stand in a refrigerator to obtain 2 g of Compound No. 14. (m.p. 130-133°C).

### Synthesis Example 5 (synthesis of Compound No. 8)

Tertiary octylbenzoquinone (10 g), 1-[4-(4-cyanophenoxycarbonylphenyl]-5-mercaptotetrazole (7 g) and a catalytic amount of p-toluenesulfonic acid were added to acetonitrile and the resulting mixture was reacted under a nitrogen stream at room temperature for 5 h. The obtained crystals were filtered and the crystals were dissolved in ethyl acetate and then washed with water and separated. After the ethyl acetate layer was dried over anhydrous sodium sulfate and concentrated, the residue was recrystallized from acetonitrile to obtain 6.5 g of Compound No. 8 (m.p.: 199-200°C).

The novel photographic DIR compound used in the photographic material of the present invention is believed to be cross-oxidized by redox reaction with the oxidized product of the developing agent formed in an image-wise pattern during development, as are the DIR hydroquinones described in U.S. Patent No. 3,379,529 and releases a development restraining substance in an image-wise pattern and is converted into a colorless oxidized product. The development restraining substance released in an image-wise pattern restrains development in an image-wise pattern and shows DIR effects such as fine-grain images, softening of the color tone, improved sharpness of the image and better color reproduction. The DIR compound used in the material of the present invention is extremely active and by even a small addition of it, produces surprisingly good effects as compared with the conventional DIR hydroquinones, as discussed later on.

The photographic DIR compound used in the material of the present invention may be used equally for a photographic material for monochrome development as well as for color image forming materials. The amounts to be used are not particularly limited, but preferably 10⁻⁶ to 1 mol/mol Ag, and particularly preferably 10⁻⁵ to 10⁻¹ mol/mol Ag are usually used.

The photographic DIR compound may be introduced into a photographic layer by dispersion by the known method which will be described later on. In this case, the photographic DIR compound may be used singly or in combination of two or more different compounds. The photographic DIR compound may also be used together with a coupler and added to the same emulsion layer as the coupler, or it may be added as a separate emulsified dispersion to a photographic auxiliary layer such as the intermediate layer.

When using the photographic DIR compound with said compound (the coupler will be described later on), the known method such as the one described in U.S. Patent No. 2,322,027 is used by introducing the compound into the silver halide emulsion layer. For example, the present compound is dissolved in the following solvents and dispersed into a hydrophilic colloid:

Alkyl phthalate (e.g., dibutyl phthalate and dioctyl phthalate), phosphate (e.g., diphenyl phosphate, triphenyl phosphate, tricresyl phosphate and dioctylbutyl phosphate), citrate (tributylacetyl citrate), benzoate (e.g., octyl benzoate), alkylamide (diethyllaurylamide), aliphatic acid esters (e.g., dibutoxyethyl succinate and diethyl azelate), trimesate (e.g., tributyl trimesate); or an organic solvent having a boiling point of 30°C to 150°C, such as lower alkyl acetates such as ethyl acetate and butyl acetat, ethyl propionate, secondary butyl alcohol, methylisobutylketone, β-ethoxyethyl acetate and methyl cellosolve acetate.

The above-illustrated high boiling point organic solvents and low boiling point organic solvents may be used in combination.

Further, the dispersion method employing the polymers described in Japanese Patent Application Publication No. 39,853/76 and Japanese Patent Application (OPI) No. 59,943/76 may also be used.

When the coupler contains an acid radical such as carboxylic acid and sulfonic acid, the present compound is introduced into a hydrophilic colloid as an aqueous alkaline solution.

Gelatin is advantageously used for the binder or protection colloid which may be used for the emulsion layer or middle layer of the photographic material of the present invention, but other hydrophilic colloids may also be employed singly or in combination with gelatin.

The gelatin may be treated with lime or an acid. The details of preparing gelatin are described in "The Macromolecular Chemistry of Gelatin" by Arthur Weiss (Academic Press, 1964).

The above-identified hydrophilic colloids which may be employed in the present invention include proteins such as a gelatin derivative, a graft polymer of a gelatin with other high polymers, albumin and casein; cellulose derivatives such as hydroxyethyl cellulose, carboxymethyl cellulose and cellulose sulfate, sugar derivatives such as sodium alginate and starch derivatives; and various synthetic hydrophilic polymer materials including homo- and copolymers such as polyvinyl alcohol, polyvinyl alcohol partially substituted by acetal, poly-N-vinyl pyrrolidone, polyacrylic acid, polymethacrylic acid, polyacrylamide, polyvinylimidazole and polyvinylpyrazole.

As the silver halide used for the photographic emulsion layer of the photographic material of the present invention, any one chosen from the group consisting of silver bromide, silver iodobromide, silver iodochlorobromide, silver chlorobromide and silver chloride may be used. The preferred silver halide is silver iodobromide containing not more than 15 mol% silver iodide.

The average size of the silver halide grains contained in the photographic emulsion is not particularly limited but preferably does not exceed 3 µm (the grain size is expressed in terms of the projected area, using as the grain size the diameter of a spherical or approximately spherical grain or the length of one edge of a cubic grain).

The grain size distribution may be either broad or narrow.

The morphology of the silver halide grains contained in the photographic emulsion may be regular such as cubes or octahedrons or may be anomalous such as spheres or tables or even a combination of these. The grains may consist of a mixture or two or more of these different morphologies.

An emulsion may be used where "super-tabular" silver halide grains having a diameter at least five times their thickness account for not less than 50% of the total projected areas.

The silver halide grains may have a surface layer whose phase is different from that of the interior. The latent images may be formed primarily on the surface of the grains or inside of them.

The photographic emulsion used in the present invention may be prepared by the methods described in "Chimie et Physique Photographique" by P. Glafkides (published by Paul Montel, 1967), "Photographic Emulsion Chemistry" by G. F. Duffin (published by The Focal Press, 1966), "Making and Coating Photographic Emulsion" by V. L Zelikman et al (published by The Focal Press, 1964) and other literature. In other words, the acid method, neutral method, ammonia method may all be used alike, and for reacting a soluble silver salt with a soluble halogen salt, the single jet method and the double jet method can be used either singly or in combination.

A method comprising forming grains in excess silver ion (the so-called reverse single jet method) may also be employed.

As one type of the double jet method, the method involving maintaining at a constant level pAg in the liquid phase wherein a silver halide is produced, i.e., the so-called controlled double jet method may be used. According to this method, the obtained silver halide emulsion has a regular crystal form and its grain size is approximately uniform.

It is also possible to use a mixture of 2 or more different silver halide emulsions prepared separately.

A cadmium salt, zinc salt, lead salt, thallium salt, iridium salt or a complex salt thereof, a rhodium salt or its complex salt, iron salt or iron complex salt may be present during the silver halide grain forming process or the physical maturing of the produced grains.

The silver halide emulsion is usually chemically sensitized. For chemical sensitization, for example, the method described in pages 675-734 of "Die Grundlagen der Photographischen Prozesse mit Silberhalogeniden" edited by H. Frieser (Akademische Verlagsgesellschaft, 1968) may be employed.

Specifically, the sulfur-sensitization employing a compound which may react with active gelatin or silver (e.g., a thiosulfate salt, thiourea, mercapto compounds and Rhodamines); the reduction sensitization employing a reducing substance (e.g., stannous salts, amines, hydrazine derivatives, formamidinesulfinate and silane compounds); and the noble metal sensitization employing noble metal compounds (e.g., complex salts, of gold and of the metals belonging to the VIII group of the Periodic Table. such as Pt, Ir and Pd). These methods may be used either singly or in combination.

Various compounds may be incorporated in the photographic emulsion used in accordance with the present invention for the purpose of preventing fog in the preparation and storage of the sensitive material or in the photographic processing or for stabilizing the photographic performance. Specifically, the following various compounds known as anti-foggants of stabilizers can be incorporated:

Azoles such as benzothiazolium salts, nitroimidazoles, nitrobenzimidazoles, chlorobenzimidazoles, bromobenzimidazoles, mercaptothiazoles, mercaptobenzothiazoles, mercaptobenzimidazoles, mercaptothiadiazoles, aminotriazoles, benzotriazoles, nitrobenzotriazoles, mercaptotetrazoles (particularly 1-phenyl-5-mercaptotetrazole); mercaptopyrimidines; mercaptotriazines; thioketo compounds such as oxazolinethione; azaindenes such as triazaindenes, tetraazaindenes (particularly 4-hydroxy-substituted (1,3,3a,7) tetraazaindenes) and pentaazaindenes; benzenethiosulfonic acid, benzenesulfinic acid, and benzenesulfonic acid amides.

The photographic emulsion layer of the photographic material of the present invention or other hydrophilic colloid layers may contain various surfactants as coating aids or antistatic agents or for providing better slip, emulsified dispersion, adhesion prevention and improved photographic properties (e.g., development acceleration, higher contrasting and sensitization).

Exemplary surfactants include the following:
nonionic surfactants such as saponin (steroid saponin), alkylene oxide derivatives (e.g., polyethylene glycol, polyethylene glycol/polyethylene glycol condensation products, polyethylene glycol alkyl ethers or polyethylene glycol alkylaryl ethers, polyethylene glycol esters, polyethylene glycol sorbitan esters, polyalkylene glycol alkylamines or polyalkylene glycol alkylamides and polyethylene oxide adducts of silicone), glycidol derivatives (e.g., alkenylsuccinic acid polyglycerides and alkylphenol polyglycerides), aliphatic acid esters of polyhydroxy alcohol and alkyl esters of sugar; anionic surfactants containing acid groups such as carboxy, sulfa and phospho groups, sulfate ester groups and phosphate ester groups, such as alkylcarboxylic acid salts, alkylsulfonic acid salts, alkylbenzenesulfonic acid salts, alkylnaphthalenesulfonic acid salts, alkylsulfate esters, alkylphosphate esters, N-acyl-N-alkyltaurines, sulfosuccinate esters, sulfoalkylpolyoxyethylenealkylphenyl ethers and polyoxyethylene alkylphosphate esters; ampholytic surface active agents such as amino acids, aminoalkylsulfonic acids, aminoalkylsulfate esters or aminoalkylphosphate esters, alkyl betaines and amine oxides; and cationic surfactants such as alkylamine salts, aliphatic or aromatic quaternary ammonium salts, heterocyclic quaternary ammonium salts such as pyridinium and imidazolium, and aliphatic or heterocycle-containing phosphonium or sulfonium salts.

In order to improve sensitivity, and contrast or to accelerate development, it is also possible to incorporate in the photographic emulsion layer of the material of the present invention, for example, polyalkylene oxides or their derivatives such as ethers, esters and amines, thioether compounds, thiomorpholines, quaternary ammonium salt compounds, urethane derivatives, urea derivatives, imidazole derivatives and 3-pyrazolidones.

In order to improve the dimensional stability and other properties, a dispersion of a synthetic polymer which does not or which hardly dissolves in water may be incorporated in the photographic emulsion layer or other hydrophilic colloid layers of the photographic material of the present invention. For example, polymers which contain as the monomeric ingredient the following substances either singly or in combination may be used:

Alkyl (meth)acrylate, alkoxyalkyl (meth)acrylate, glycidyl (meth)acrylate, (meth)acrylamide, vinyl esters (e.g. vinyl acetate), acrylonitrile, olefin and styrene. Further, these may be used in combination with acrylic acid, methacrylic acid, α,β-unsaturated dicarboxylic acid, hydroxyalkyl (meth)acrylate, sulfoalkyl (meth)acrylate and styrene sulfonic acid.

The photographic emulsion used in the present invention may be spectrally sensitized, for example, with methine dyes. Exemplary dyes used in the present invention include cyanine, merocyanine, complex merocyanine, horopolar cyanine, hemicyanine, styryl and hemioxonol dyes. Particularly useful dyes are cyanine and merocyanine dyes and those dyes which belong to the group of complex merocyanine dyes. Any one of the nuclei generally used for cyanine dyes as basic heterocyclic nuclei is applicable for the above-illustrated dyes. Specifically, pyrroline, oxazoline, thiazoline, pyrrole, oxazole, thiazole, selenazole, imidazole, tetrazole and pyridine nuclei; nuclei formed by coalescence of alicyclic hydrocarbon rings to these nuclei; and nuclei formed by fusion of aromatic hydrocarbon rings to the above-illustrated nuclei, such as indolenine, benzindolenine, indole, benzoxazole, naphthooxazole, benzothiazole, naphthothiazole, benzoselenazole, benzimidazole and quinoline nuclei. These nuclei may be substituted on carbon atoms.

The following 5- to 6-membered heterocyclic nuclei may be applicable as nuclei having the ketomethylene structure for merocyanine dyes or compound merocyanine dye: pyrazoline-5-one nucleus, thiohydantoin nucleus, 2-thiooxazolidine-2,4-dione nucleus, thiazolidine-2,4-dione nucleus, Rhodamine nucleus and thiobarbituric acid nucleus.

These sensitizing dyes may be used either singly or in combination. In particular, combinations of these sensitizing dyes are frequently used for super sensitization.

Aside from the above-identified sensitizing dyes, it is also possible to incorporate in the emulsion dyes which have no spectral sensitization effects or substances which are substantially incapable of absorbing visible rays and which show the super sensitization ability. Exemplary substances which may be thus incorporated include aminostilbene compounds substituted with nitrogen-containing heterocyclic groups (e.g., those compounds described in U.S. Patents Nos. 2,933,390 and 3,635,721), aromatic organic acid formaldehyde condensation products (e.g., those compounds described in U.S. Patent No. 3,743,510), cadmium salts and azaindene compounds.

The present invention may be applied to multilayer multicolor photographic materials having at least two different degrees of spectral sensitivity on the substrate. Multilayer natural color photographic materials usually have on the substrate at least one each of red-sensitive, green-sensitive and blue-sensitive emulsion layers. The order of these layers may be selected optionally as required. Generally cyanogen-forming, magenta-forming and yellow-forming couplers are incorporated into the red-sensitive, green-sensitive and blue-sensitive emulsion layers, respectively, but different combinations are possible in some cases.

In the photographic material of the present invention, the photographic emulsion layer or non-sensitive layers can contain, in addition to the above-illustrated compounds other dye-forming couplers, i.e., compounds which may develop colors by oxidative coupling with aromatic primary amine developing agents (e.g., phenylenediamine derivatives and aminophenol derivatives) in the color developing process. Exemplary conventional magenta couplers include 5-pyrazolone couplers, pyrazolobenzimidazole couplers, pyrazoloimidazol couplers, pyrazolopyrazole couplers, pyrazolotriazole couplers, pyrazolotetrazole couplers, cyanoacetylcoumarone couplers and open chain acylacetonitrile couplers. Exemplary conventional yellow couplers include acylacetamide couplers (e.g., benzoylacetanilides and pivaloylacetanilides). Exemplary conventional cyanogen couplers include naphthol couplers and phenol couplers. These couplers are desirably of a non diffusive type and contain a hydrophobic group called a ballast group in the molecule. The couplers may also be in the form of polymers. The couplers may be either four- or two-equivalent with respect to silver ion. The copulers may also be colored couplers having color correction effects or couplers which release development restrainers in the course of development (i.e., the so-called DIR couplers).

In addition to the DIR couplers, colorless DIR coupling compounds which produce colorless substances by a coupling reaction and which release development restrainers may also be incorporated in the above-identified layers. Aside from DIR couplers, compounds which release development restrainers in the course of development may be incorporated in the sensitive material.

In order to meet the requirements for a sensitive material, two or more different kinds of the above-illustrated couplers may be used in the same layer of the photographic material of the present invention. Needless to say, the same compound may be added to two or more different layers of the photographic material of the present invention.

Inorganic or organic hardeners may be incorporated in the hydrophilic colloid layers such as the photographic emulsion layer. For example, the following substances may be used either singly or in combination:

Chromium salts (e.g., chromium alum and chromium acetate), aldehydes (e.g., formaldehyde, glyoxal and glutaraldehyde), N-methylol compounds (e.g., dimethylol urea and methylol dimethylhydantoin), dioxane derivatives (e.g., 2,3-dihydroxydioxane), active vinyl compounds (e.g., 1,3,5-triacryloyl-hexahydro-s-triazine and 1,3-vinylsulfonyl-2-propanol), active halides (e.g., 2,4-dichlor-6-hydroxy-s-triazine) and mucohalogenic acids (e.g., mucochloric acid and mucophenoxychloric acid).

If dyes or ultraviolet ray absorbing agents are incorporated in the hydrophilic colloid layers of the photographic material of the present invention, the dyes or the UV ray absorbing agents may be mordanted, for example, with cationic polymers.

The sensitive material of the present invention may contain as an anti-color-fogging agent derivatives of hydroquinone, aminophenol, gallic acid and ascorbic acid.

The photographic material of the present invention may contain an ultraviolet ray absorbing agent in the hydrophilic colloid layer. Exemplary compounds to be used include benzotriazole compounds substituted with an aryl group (e.g., those described in U.S. Patent No. 3,533,794), 4-thiazolidone compounds (e.g., those described in U.S. Patents 3,314,794 and 3,352,681), benzophenone compounds (e.g., those described in Japanese Patent Application (OPI) No. 2,784/71), cinnamic acid ester compounds (e.g., those described in U.S. Patents Nos. 3,705,805 and 3,707,375), butadiene compounds (e.g., those described in U.S. Patent No. 4,045,229) and benzooxidol compounds (e.g., those described in U.S. Patent No. 3,700,455). An ultraviolet ray absorbing coupler (e.g., cyanogen dye forming coupler of α-naphthol) or an ultraviolet ray absorbing polymer may also be used. These ultraviolet ray absorbing agents may be mordanted in specific layers.

The sensitive material of the present invention may contain a water-soluble dye as a filter dye or for various purposes such as prevention of irradiation in the hydrophilic colloid layer. Exemplary dyes to be used include oxonol, hemioxonol, styryl, merocyanine, cyanine and azo dyes. Among these, oxonol dyes, hemioxonol dyes and merocyanine dyes are particularly useful.

The following known anti-fading agents may be incorporated in the photographic material, and the color image stabilizers used in the present invention may be employed either singly or in combination. Known anti-fading agents include hydroquinone derivatives, gallic acid derivatives, p-alkoxyphenols, p-oxyphenol derivatives and bisphenols.

In the photographic processing of the silver halide photographic material of the present invention, any suitable known method can be employed, utilizing any known processing solution. The processing temperature is usually chosen within the range of 18°C to 50°C, but temperatures lower than 18°C or higher than 50°C may also be employed. Either of the monochrome development process wherein a silver image is formed in accordance with specific purposes and the color photographic development process wherein dye images are formed may be applied for the silver halide photographic material of the present invention.

The developer solution used in monochrome development may contain a conventionally known developing agent. Exemplary developing agents which may be employed either singly or in combination include:

Dihydroxybenzenes (e.g., hydroquinone), 3-pyrazolidones (e.g., 1-phenyl-3-pyrazolidone), aminophenols (e.g., N-methyl-p-aminophenol), 1-phenyl-3-pyrazolines, ascorbic acid, and heterocyclic compounds produced by condensation of 1,2,3,4-tetrahydroquinoline rings and indolene rings, such as those described in U.S. Patent No. 4,067,872. In addition to these compounds, the developer solution generally contains a known preservative, an alkaline agent, a pH buffer, and an anti-fogging agent, and may contain, as required, a solubilizer, a color toning agent, a development accelerator, a surfactant, an antifoaming agent, a water softener, a hardener, and a tackifier.

As the fixing solution, the commonly used composition may be employed. Exemplary fixing agents include thiosulfate, thiocyanate, and organic sulfur compounds which are known to have fixing effects. The fixing solution may contain a water-soluble aluminum salt as the hardener.

Conventional methods may be employed for forming dye images. Exemplary methods include 1) the negative-positive process (see "Journal of the Society of Motion Picture and Television Engineers, vol. 61, pp. 667-701, 1973), 2) the color reversal process comprising forming negative silver images by developing a film in a developer solution containing a black and white developing agent and performing a suitable anti-fogging treatment such as at least one cycle of uniform exposure, followed by color development to form positive dye images, and 3) the silver dye bleach process comprising subjecting the photographic emulsion layer containing dyes to exposure followed by development to form silver images and then bleaching the dyes using the silver images as the bleaching catalyst.

The color developer solution generally consists of an aqueous alkaline solution containing a color developing agent. Exemplary conventional color developing agents to be used in the material of the present invention include known primary aromatic amine developing agents such as phenylenediamines (e.g., 4-amino-N,N-diethylaniline, 3-methyl-4-amino-N,N-diethylaniline, 4-amino-N-ethyl-N-β-hydroxyethylaniline, 3-methyl-4-amino-N-ethyl-N-β-hydroxyethylaniline, 3-methyl-4-amino-N-ethyl-N-β-methanesulfonamidoethylaniline or 4-amino-3-methyl-N-ethyl-N-β-methoxyethylaniline).

In addition to the above-illustrated developing agents, those compounds described on pp. 226-229 of "Photographic Processing Chemistry" by L F. A. Mason (published by Focal Press, 1966), U.S. Patents Nos. 2,193,015 and 2,592,364 and Japanese Patent Application (OPI) No. 64,933/73, may also be employed.

The color developer solution may, in addition to the above-listed compounds, contain a pH buffer such as sulfite, carbonate, borate and phosphate of alkali metals, a development restrainer or anti-fogging agent such as bromide, iodide and an organic anti-fogging agent. The development solution may also, as required, contain a water softener, a preservative such as hydroxylamine, an organic solvent such as benzyl alcohol and diethylene glycol, a development accelerator such as polyethylene glycol, quaternary ammonium salts and amines, a dye forming coupler, a competitive coupler, a fogging agent such as sodium borohydride, an auxiliary developing agent such as 1-phenyl-3-pyrazolidone, a tackifier, polycarboxylic acid compounds used as chelate agents as described in U.S. Patent No. 4,083,723, and an anti-oxidizing agent as described in West German Offenlegungsschrift (OLS) No. 2,622,950.

The photographic emulsion layer is usually subjected to bleaching after completion of the color development process. The bleach process may or may not be performed simultaneously with the fixation process. Exemplary bleaching agents include compounds of polyvalent metals such as iron (II), cobalt (III), chromium (VI) and copper (II), peracids, quinones and nitroso compounds. Specifically, ferricyanide, dichromate, organic complex salts of iron (III) or cobalt (III), complex salts of aminopolycarboxylic acids such as ethylenediamine tetraacetic acid, nitrilotriacetic acid and 1,3-diamino-2-propanol tetraacetic acid, or of organic acids such as citric acid, tartaric acid and malic acid; persulfate and permanganate; and nitrosophenol. Among these particularly useful are potassium ferricyanide EDTA iron (III) sodium and EDTA iron (III) ammonium. Complex salts of EDTA iron (III) are useful both in an independent bleaching solution and a combined bleaching and fixing solution.

Various additives may be added to the bleaching or bleaching and fixing solution, in addition to the bleaching accelerators as described in U.S. Patents Nos. 3,042,520 and 3,241,966, and Japanese Patent Application Publication Nos. 8,506/70 and 8,836/70, and the thiol compounds as described in Japanese Patent Application (OPI) No. 65,732/78.

The photographic DIR compound used in the present invention may be used for various silver halide photographic materials. It is useful for both the black and white photography and the color photography. The compound used in the present invention may also be employed for various silver halide photographic materials used for the general black and white photography, monochrome printing, X-ray, micro lithography with electron beams, high resolution black and white photography, diffusion transfer black and white photography, general color photography, color X-ray, and diffusion transfer color photography.

The DIR compound used in the present invention is highly active and quickly releases a development restrainer by oxidation. Because of this advantage, addition of only a small amount of this compound produces DIR effects such as the tone control, finer-grain images, increased sharpness and better color reproduction. Further, the DIR compound used in the present invention is stable in the silver halide photographic material and does not cause deterioration of shelf stability of the photographic material.

The DIR compound used in the present invention is free from any accumulation of a development restrainer in the developer solution which may adversely affect the development process when the photographic material is processed in large quantities. Thus, the desired DIR effects may be obtained.

The present invention will now be further described in detail by reference to the following examples.

### Example 1

A multilayer color photographic material comprising layers of the following compositions was prepared on a cellulose triacetate film substrate.
- First layer:: antihalation layer (AHL)
a gelatin layer containing black colloidal silver
- Second layer:: middle layer
a gelatin layer containing an emulsified dispersion of 2,5-di-t-octylhydroquinone
- Third layer:: first red sensitive emulsion layer (RL₁)
Silver iodobromide emulsion (silver iodobromide: 5 mol%): amount of silver coated:
1.79 g/m²
Sensitizing dye I: 6 × 10⁻⁵ mol per mol of silver
Sensitizing dye II: 1.5 × 10⁻⁵ mol per mol of silver
Coupler A: 0.04 mol per mol of silver
Coupler C-1: 0.0015 mol per mol of silver
Coupler C-2: 0.0015 mol per mol of silver
Above-identified Compound No. 3: 0.0006 mol per mol of silver
- Fourth layer:: second red sensitive emulsion layer (RL₂)
Silver iodobromide emulsion (silver iodide: 4 mol%): amount of silver coated: 1.4 g/m²
Sensitizing dye I: 3 × 10⁻⁵ mol per mol of silver
Sensitizing dye II: 1.2 × 10⁻⁵ mol per mol of silver
Coupler A: 0.005 mol per mol of silver
Coupler C-1: 0.0008 mol per mol of silver
Coupler C-2: 0.0008 mol per mol of silver
Above-identified Compound No. 3: 0.00006 mol per mol of silver
- Fifth layer:: middle layer (ML)
Same as the second layer
- Sixth layer:: first green sensitive emulsion layer (GL₁)
Silver iodobromide emulsion (silver iodide: 4 mol%): amount of silver coated: 1.5 g/m²
Sensitizing dye III: 3 × 10⁻⁵ mol per mol of silver
Sensitizing dye IV: 1 × 10⁻⁵ mol per mol of silver
Coupler B: 0.05 mol per mol of silver
Coupler M-1: 0.008 mol per mol of silver
Above-identified Compound No. 3: 0.0015 mol per mol of silver
- Seventh layer:: second green sensitive emulsion layer (GL₂)
Silver iodobromide emulsion (silver iodide: 5 mol%): amount of silver coated: 1.6 g/m²
Sensitizing dye III: 2.5 x 10⁻⁵ mol per mol of silver
Sensitizing dye IV: 0.8 x 10⁻⁵ mol per mol of silver
Coupler B: 0.02 mol per mol of silver
Coupler M-1: 0.003 mol per mol of silver
Above-identified Compound No. 3: 0.0003 mol per mol of silver
- Eighth layer:: yellow filter layer (YEL)
A gelatin layer containing in the aqueous gelatin solution an emulsified dispersion of yellow colloidal silver and 2,5-di-t-octylhydroquinone
- Ninth layer:: first blue sensitive emulsion layer (BL₁)
Silver iodobromide emulsion (silver iodide: 6 mol%.): amount of silver coated: 1.5 g/m²
Coupler Y-1: 0.25 mol per mol of silver
- Tenth layer:: second blue sensitive emulsion layer (BL₂)
Silver iodobromide emulsion (silver iodide: 6 mol%.): amount of silver coated: 1.1 g/m²
Coupler Y-1: 0.06 mol per mol of silver
- Eleventh layer:: protective layer (PL)
Coating of a gelatin layer containing polymethyl methacrylate particles (diameter: about 1.5 µm).

In addition to the above-identified ingredients, a gelatin hardener and a surfactant were added to the layers.

The sample prepared as above was named Sample No. 101.

Sample No. 102: This sample was prepared in the same manner as Sample No. 101 except that
1) Compound No. 3 added to RL₁ and RL₂ was replaced by an equal amount of Compound No. 7, and
2) Compound No. 3 added to GL, and GL2 was replaced by Compound No. 14 which was used in an amount twice that of Compound No. 3.

Sample No. 103: This sample was prepared in the same manner as Sample No. 101 except that Compound No. 3 of Sample No. 101 was replaced by an equal amount of a DIR compound (DIR-1).

Sample No. 104: This sample was prepared in the same manner as Sample No. 101 except that Compound No. 3 of Sample No. 101 was replaced by an equal amount of a DIR compound (DIR-2).

DIR compounds used as control 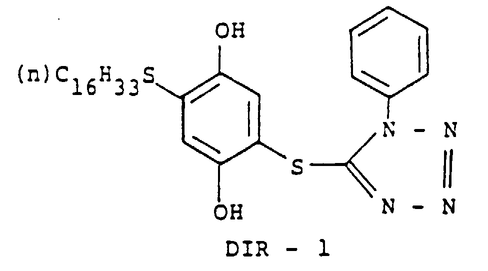 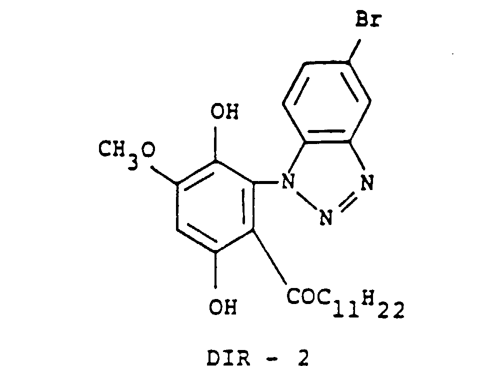 Compounds used for preparation of the samples
- Sensitizing dye I:: pyridinium salt of anhydro-5,5'-dichloro-3,3'-di-(γ-sulfopropyl)-9-ethyl-thiacarbocyaninehydroxide.
- Sensitizing dye II:: triethylamine salt of anhydro-9-ethyl-3,3'-di-(γ-sulfopropyl)-4,5,4',5'-dibenzothiacarbocyaninehydroxide.
- Sensitizing dye III:: sodium salt of anhydro-9-ethyl-5,5'-dichloro-3,3'-di-(y-sulfopropyl)-oxacarbocyanine.
- Sensitizing dye IV:: sodium salt of anhydro-5,6,5',6'-tetrachloro-1,1'-diethyl-3,3'-di-β-[β-(y-sulfopropoxy)ethoxy)ethylimidazolocarbocyaninehydroxide.

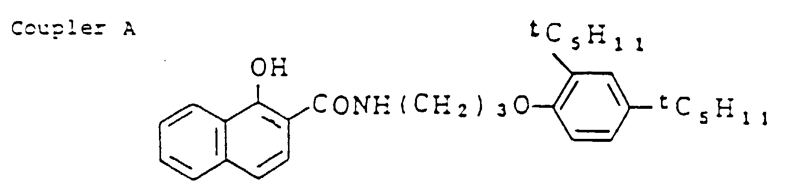

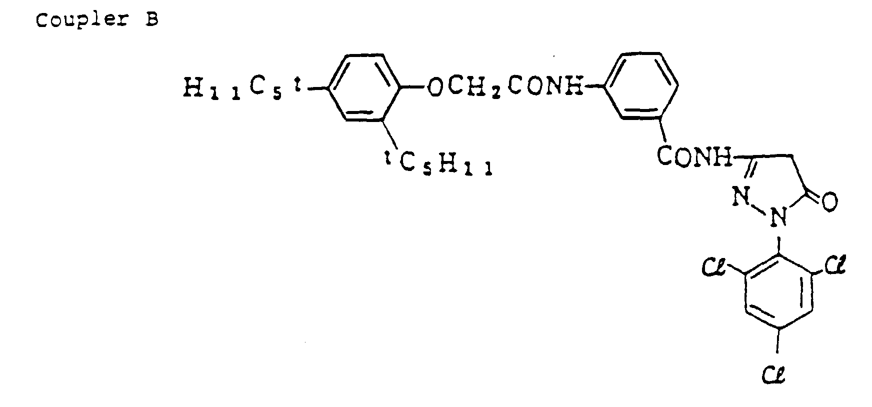

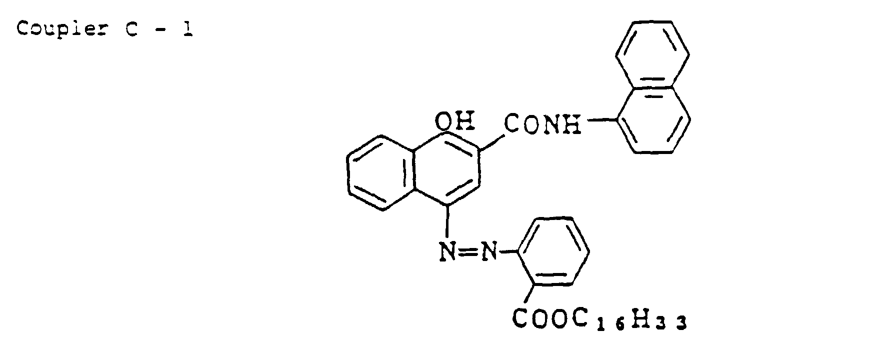

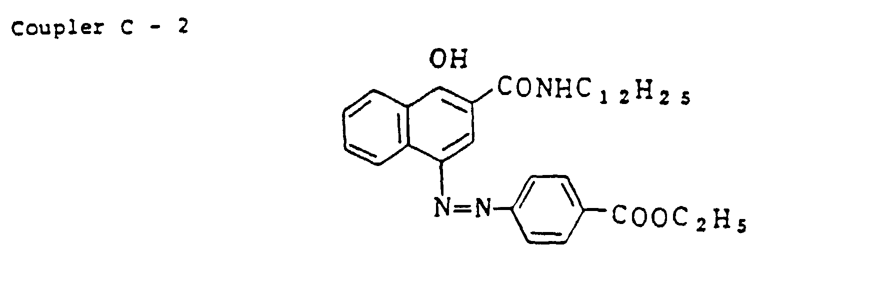

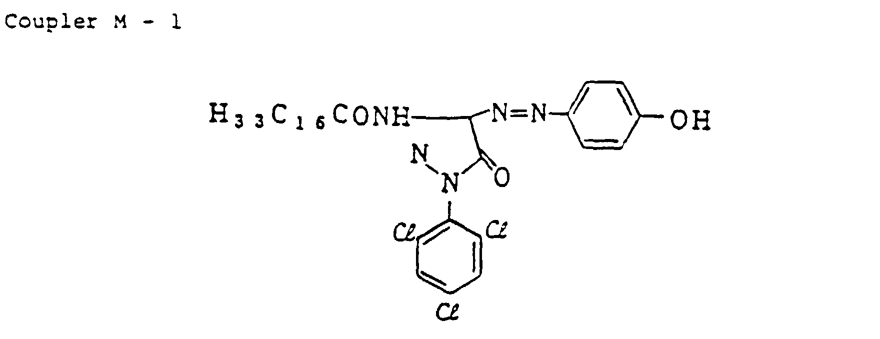

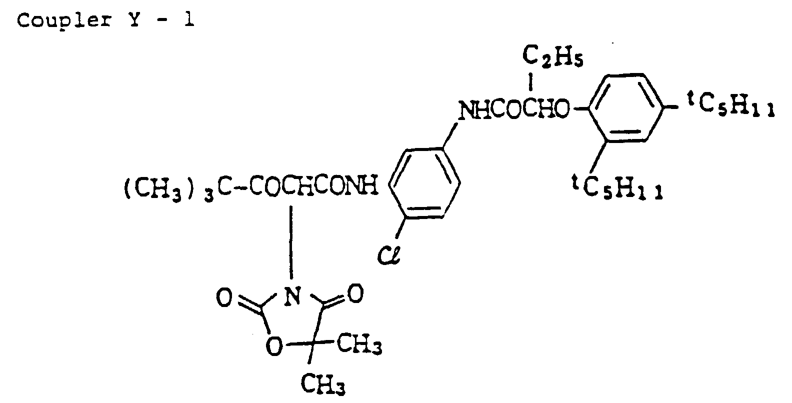

The obtained Samples Nos. 101 to 104 were made into 35 mm size films, followed by wedge exposure. A 600 m portion of each of these films was subjected to the following development process in a 2 l developer solution tank.

| | |
|---|---|
| 1. Color development | 3 min 15 s |
| 2. Bleach | 6 min 30 s |
| 3. Rinsing | 3 min 15 s |
| 4. Fixation | 6 min 30s |
| 5. Rinsing | 3 min 15 s |
| 6. Stabilisation | 3 min 15 s |

The compositions of the processing solutions used for the above steps are shown below:

### Colour development solution

| | |
|---|---|
| Sodium nitrilotriacetate | 1.0 g |
| Sodium sulfite | 4.0 g |
| Sodium carbonate | 30.0 g |
| Potassium bromide | 1.4 g |
| Hydroxylamine sulfate | 2.4 g |
| 4-(N-ethyl-N-β-hydroxyethylamino)-2-methyl-aniline sulfate | 4.5 g |
| Water to make | 1 l |

### Bleaching solution

| | |
|---|---|
| Ammonium bromide | 160.0 g |
| Aqueous ammonia (28%) | 25.0 ml |
| EDTA sodium iron salt | 130.0 g |
| Glacial acetic acid | 14 ml |
| Water to make | 1 l |

### Fixing solution

| | |
|---|---|
| Sodium tetrapolyphosphate | 20. g |
| Sodium sulfite | 4.0 g |
| Ammonium thiosulfate (70%) | 175.0 ml |
| Sodium bisulfite | 4.6 g |
| Water to make | 1 l |

### Stabilizing solution

| | |
|---|---|
| Formalin | 8.0 ml |
| Water to make | 1 l |

The overflow portion of the developing solution was subjected to the following recycle treatment for repeated reuse.

The recycle treatment was performed batch-wise. The overflow solution was introduced into an electric dialyzer and electric dialysis was performed until the amount of KBr became not greater than 0.7 g/l.

To this solution were added sodium nitrilotriacetate, sodium sulfite, sodium carbonate, potassium bromide, hydroxylamine sulfate and 4-(N-ethyl-N-β-hydroxyethylamino)-2-methyl-aniline sulfate, which were all consumed in the running. The mixture was subjected to pH adjustment to a final pH of 10.05 and the resultant solution was reused as a replenisher. Table 1 shows the decreases in sensitivity after 10 cycles of reuse (1 l of the overflow solution was used for 1 resue cycle).

As is clear from the results shown in Table 1, Samples Nos. 101, and 102, showed no substantial sensitivity decrease, whereas Samples Nos. 103 and 104 suffered a large drop in sensitivity. This result shows that the groups which left Compounds Nos. 3, 7, 14, 58 and 72 form, in the color developing solution, compounds which have a photographic effects, and are not accumulated in the developer solution, as in the case of other non-deactivating type leaving groups, making repeated reuse of the solution possible.

**Table 1**

| *ΔSfog + 0.3 | | | |
|---|---|---|---|
| Sample No. | B | G | R |
| 101 | +0.02 | ±0 | ±0 |
| 102 | +0.03 | -0.02 | ±0 |
| 103 | -0.21 | -0.13 | -0.06 |
| 104 | -0.16 | -0.07 | ±0 |

| | | | |
|---|---|---|---|
| * The sensitivity drop at (fog + 0.3) density is expressed by log E. | | | |

### Example 2

Sample No. 201: This sample was prepared in the same manner as Sample No. 101 except that compound No. 3 for Sample No. 101 was replaced by Compound No. 5 which was used in an amount 1.2 times as much as that of Sample No. 3.

Sample No. 202: This sample was prepared in the same manner as Sample NO. 101 except that Compound No. 3 of Sample No. 101 was replaced by Compound No. 15 which was used in an amount twice that of Sample No. 3.

Sample No. 203: This sample was prepared in the same manner as Sample No. 101 except that Compound No. 3 of Sample No. 101 was replaced by an equal amount of a DIR compound (DIR-3).

DIR compound (DIR-3) used as control.

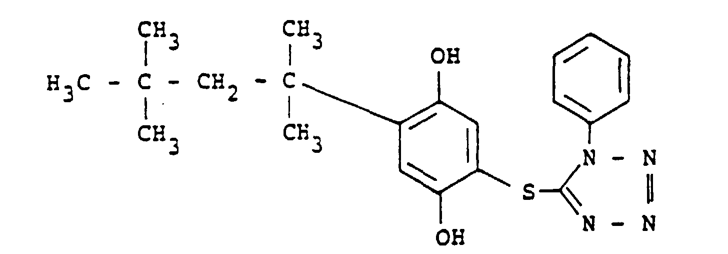

Each of Samples Nos. 201 to 203 was made into a 35 mm film, and this film was subjected to wedge exposure. Then a 100 m portion of each of the films was subjected to the same development procedure as that of Example No. 1 in a tank containing 5 1 of the developing solution. The sensitivity of the first portion of each of the processed samples was compared with that of the end portion, and the decrease in sensitivity of the end portion of each of the samples is shown in Table 2.

As is clear from the results shown in Table 2, Samples Nos. 201 and 202 suffer from a smaller sensitivity drop than Sample No. 203. This means that the groups which left Compounds Nos. 5 and 15 were converted in the color developing solution into compounds which have no substantial photographic effects.

**Table 2**

| *ΔSfog + 0.3 | | | |
|---|---|---|---|
| Sample No. | B | G | R |
| 201 | +0.01 | ±0 | ±0 |
| 202 | +0.03 | +0.01 | ±0 |
| 203 | -0.22 | -0.14 | -0.07 |

| | | | |
|---|---|---|---|
| * The sensitivity drop at (fog + 0.3) density is expressed by log E. | | | |

### Example 3

A silver halide emulsion comprising 80 mol% silver chloride, 19.5 mol% siler bromide and 0.5 mol% silver iodide was prepared by gold sensitization and sulfur sensitization according to the conventional method. The gelatin contained in this emulsion was 45% based on the weight of the silver halide. To this emulsion were added potassium salt of 5 - [3 - (δ sulfobutyl) - 5 - chloro - 2 - oxazolidilidene-ethylidene) - 1 - hydroxyethoxyethyl - 3 - (2 - pyridyl) - 2 - thiohydantoin (spectral sensitizer), sodium dodecylbenzenesulfonate (surfactant) and the polymer latex described in Formulation No. 3 of a preparation example of U.S. Patent No. 3,535,620. Then, 1,2-bis(vinylsulfonylacetamido)ethane (hardener) was added to the resulting mixture to a final ratio of 2.6% based on the total dry weight of the gelatin used in the composition (i.e., the total dry gelatin including that which was added to the non-photosensitive upper layer which will be described below). Finally, the compound used in the present invention was added to the resulting mixture in the form of a methanol solution as shown in Table 3 to obtain a coating solution for a photographic silver halide emulsion layer.

On the other hand, a coating solution for the non-sensitive upper layer was prepared separately by adding to a 5% gelatin solution sodium dodecylbenzenesulfonate (surfactant) and a polymethylmethacrylate latex (matting agent) having an average grain size of 3.0 to 4.0 µm.

Then, the above-identified coating solution for the silver halide emulsion layer and the coating solution for the non-sensitive upper layer were applied to a polyethylene terephthalate substrate by the two-coat simultaneous coating method. The amount of silver coated was 3.0 g/m², and the thickness of the dried film of the non-sensitive upper layer was 1.0 µm.

These samples were exposed to white tungsten light for 8 s through an optical step wedge having a density difference of 0.1 between two adjacent steps.

Then, half-tone images were formed on these samples by the following method:

A commercial negative grey contact screen (150 lines/2,54 cm) was brought into close contact with the samples and the samples were exposed to white tungsten light for 10 s through an optical step wedge having a density difference of 0.1.

The above-identified exposed samples were developed in the following developing solution at 38°C for 20 s, followed by fixation, rinsing and drying by the conventional method.

### Composition of the developing solution

| | |
|---|---|
| Sodium sulfite | 75 g |
| Sodium hydrogencarbonate | 7 g |
| Hydroquinone | 40 g |
| 1-phenyl-4,4-dimethyl-3-pyrazolidone | 0.4 g |
| Sodium bromide | 3 g |
| 5-methylbenzotriazole | 0.8 g |
| Ethylenediamine tetraacetic acid sodium salt | 1 g |
| 3-diethylamino-1,2-propanediol | 20 g |
| Water to make | 1 l |
| Adjustment to pH = 11.4 | |

The relative sensitivity and the results of γ evaluation are shown in Table 3.

**Table 3**

| Sample No. | Compound | Amount added | Relative sensitivity* | γ |
|---|---|---|---|---|
| 301 | - | - | 100 | 5.2 |
| 302 | (7) | 3.0 x 10⁻⁴ mol/mol Ag | 90 | 3.5 |

| | | | | |
|---|---|---|---|---|
| * The relative sensitivity was determined with a density of 1.5 taken as 100. | | | | |

As is clear from Table 3, the compound used in the present invention provides the desired DIR effects and particularly the tone softening effect.

### Example 4

Sample No. 307 was prepared in the same manner as Sample No. 302 except that compound No. 7 for Sample No. 302 of Example 3 was replaced by an equal amount of a DIR compound (DIR-1), and this Sample No. 307 as used for comparison purposes.

Each of the Samples was made into 3 x 15 cm sample film portions. One thousand sample portions of each of Samples Nos. 301, 302 and 307 were subjected to the same developing procedure of Example 3, and the sensitivity of the first portion of each of the processed samples was compared with that of the end portion. The results are shown in Table 4.

**Table 4**

| Sample No. | Compound | Relative sensitivity* |
|---|---|---|
| 301 | - | 100 |
| 302 | (7) | 97 |
| 307 | DIR-1 | 75 |

| | | |
|---|---|---|
| * Relative sensitivity was compared at a density of 0.2 | | |

As is clear from these results, Sample No. 302 containing the compounds used in the present invention suffer from smaller sensitivity drops than Sample No. 307. This means that the compounds used in the present invention, after being dissolved in the developer solution, are converted into compounds which have no substantial effects on the photographic processing.

## Claims

1. A silver halide photographic material containing at least one compound having a group which after being released from the compound by redox reaction is converted into a compound having a development restraining ability and which is further converted in the developing solution into a compound which has no substantial development restraining ability or which shows a marked decrease in such ability wherein said compound is represented by the general formula (I): 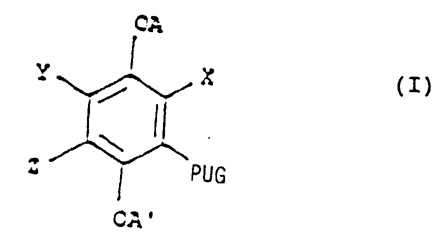 wherein
A and A' are each hydrogen or a group which can be hydrolyzed by alkali;
X, Y and Z are each hydrogen, a halogen atom, a substituted or unsubstituted alkyl group, a substituted or unsubstituted alkoxy group, a substituted or unsubstituted aryl group or an alkylthio group, X may additionally be an electron-withdrawing group selected from 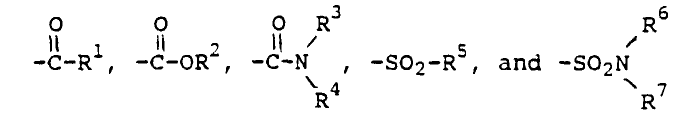
wherein R¹ to R⁷ are each a substituted or unsubstituted alkyl group, a substituted or unsubstituted aryl group or a substituted or unsubstituted aralkyl group;
or Y and Z may form a 5- or 6-membered ring through a methylene chain;
PUG is a group released by reaction with a light-exposed silver halide and/or the oxidized product of the developing agent and is bonded to the benzene nucleus via a sulfur atom or a nitrogen atom,
wherein said PUG is selected from the group consisting of formulae (P-1) and (P-2): 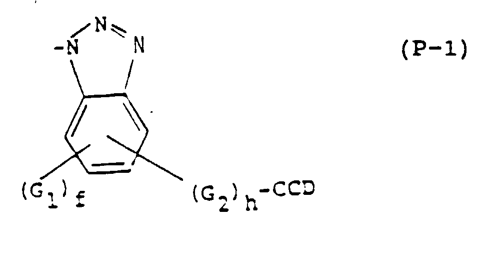 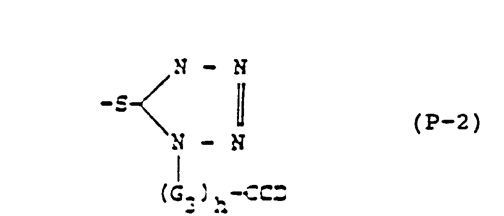
wherein
G₁ is hydrogen, a halogen atom, an alkyl group, an acylamino group, an alkoxy group, a sulfonamido group, an aryl group, an alkylthio group, an alkylamino group, an anilino group, an amino group, an alkoxycarbonyl group, an acyloxy group, a nitro group, a cyano group, a sulfonyl group, an aryloxy group, a hydroxy group, a thioamido group, a carbamoyl group, a sulfamoyl group, a carboxyl group, a ureido group or an aryloxycarbonyl group;
G₂ is any one of the substituents listed for G₁ which may form a divalent group;
G₃ is a substituted or unsubstituted alkylene group or a substituted or unsubstituted arylene group and may be interrupted by an ether, ester, thioether, amido, ureido, imido, sulfon or sulfonamido bond or a carbonyl group, wherein said bonding groups and alkylene and arylene groups may be linked together to form a divalent group;
f is an integer of 1 or 2;
h is 0 or 1, and
CCD is
-COOR⁸ (D-1)
wherein
R⁸ is a substituted or unsubstituted alkyl group, a substituted or unsubstituted aryl group or a substituted or unsubstituted aralkyl group.

2. The silver halide photographic material of claim 1 wherein said compound is employed in an amount of from 10⁻⁶ to 1 mol/mol of silver.

3. The silver halide photographic material of claim 1 wherein said compound is employed in an amount of from 10⁻⁵ to 10⁻¹ mol/mol of silver.

## Patentansprüche

1. Photographisches Silberhalogenidmaterial, enthaltend wenigstens eine Verbindung mit einer Gruppe, die nach der Freisetzung aus der Verbindung durch Redoxreaktion in eine Verbindung mit einem Entwicklungsverzögerungsvermögen umgewandelt wird und die weiterhin in der Entwicklungslösung in eine Verbindung umgewandelt wird, die im wesentlichen kein Entwicklungsverzögerungsvermögen oder die eine bemerkenswerte Erniedrigung in diesem Vermögen zeigt, worin die Verbindung durch die allgemeine Formel (I): 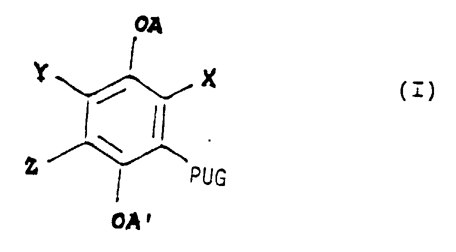 dargestellt wird, worin
A und A' jeweils Wasserstoff oder eine Gruppe, die durch Alkali hydrolysiert werden kann, ist;
X, Y und Z jeweils Wasserstoff, ein Halogenatom, eine substituierte oder unsubstituierte Alkylgruppe, eine substituierte oder unsubstituierte Alkoxygruppe, eine substituierte oder unsubstituierte Arylgruppe oder eine Alkylthiogruppe sind, X zusätzlich eine elektronen-abziehende Gruppe ausgewählt aus 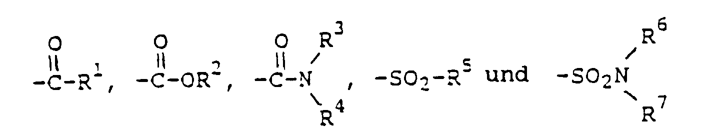
sein kann, worin R¹ bis R⁷ jeweils eine substituierte oder unsubstituierte Alkylgruppe, eine substituierte oder unsubstituierte Arylgruppe oder eine substituierte oder unsubstituierte Aralkylgruppe sind;
oder Y und Z einen 5- oder 6-gliedrigen Ring durch eine Methylenkette bilden können;
PUG eine Gruppe ist, die durch Reaktion mit einem belichteten Silberhalogenid und/oder dem oxidierten Produkt des Entwicklungsmittels freigesetzt wird und an den Benzolkern über ein Schwefelatom oder ein Stickstoffatom gebunden ist,
worin PUG aus der Gruppe, bestehend aus den Formeln (P-1) und (P-2): 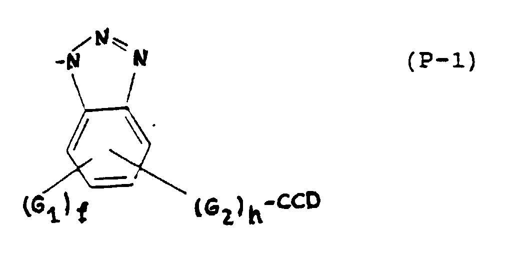 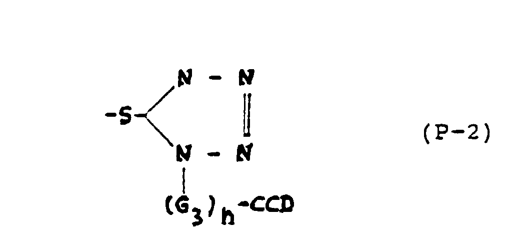 gewählt wird, worin
G₁ Wasserstoff, ein Halogenatom, eine Alkylgruppe, eine Acylaminogruppe, eine Alkoxygruppe, eine Sulfonamidogruppe, eine Arylgruppe, eine Alkylthiogruppe, eine Alkylaminogruppe, eine Anilinogruppe, eine Aminogruppe, eine Alkoxycarbonylgruppe, eine Acyloxygruppe, eine Nitrogruppe, eine Cyanogruppe, eine Sulfonylgruppe, eine Aryloxygruppe, eine Hydroxygruppe, eine Thioamidogruppe, eine Carbamoylgruppe, eine Sulfamoylgruppe, eine Carboxylgruppe, eine Ureidogruppe oder eine Aryloxycarbonylgruppe ist;
G₂ einer der für G₁ angegebenen Substituenten ist, der eine zweiwertige Gruppe bilden kann;
G₃ eine substituierte oder unsubstituierte Alkylengruppe oder eine substituierte oder unsubstituierte Arylengruppe ist und durch eine Ether-, Ester-, Thioether-, Amido-, Ureido-, Imido-, Sulfon- oder Sulfonamidobindung oder eine Carbonylgruppe unterbrochen sein kann, worin die Bindungsgruppen und die Alkylen- und Arylengruppen miteinander zur Bildung einer zweiwertigen Gruppe verbunden sein können;
f eine ganze Zahl von 1 oder 2 ist;
h 0 oder 1 ist, und
CCD
-COOR⁸ (D-1)
ist,
worin R⁸ eine substituierte oder unsubstituierte Alkylgruppe, eine substituierte oder unsubstituierte Arylgruppe oder eine substituierte oder unsubstituierte Aralkylgruppe ist.

2. Photographisches Silberhalogenidmaterial nach Anspruch 1, worin die Verbindung in einer Menge von 10⁻⁶ bis 1 Mol/Mol Silber verwendet wird.

3. Photographisches Silberhalogenidmaterial nach Anspruch 1, worin die Verbindung in einer Menge von 10⁻⁵ bis 10⁻¹ Mol/Mol Silber verwendet wird.

## Revendications

1. Matériau photographique à l'halogénure d'argent contenant au moins un composé ayant un groupe qui, après avoir été libéré du composé par une réaction rédox, est converti en un composé ayant une aptitude à limiter le développement et qui est en outre converti dans la solution de développement en un composé qui n'a aucune aptitude sensible à limiter le développement ou qui manifeste une diminution marquée de cette aptitude, dans lequel ledit composé est représenté par la formule générale (I): 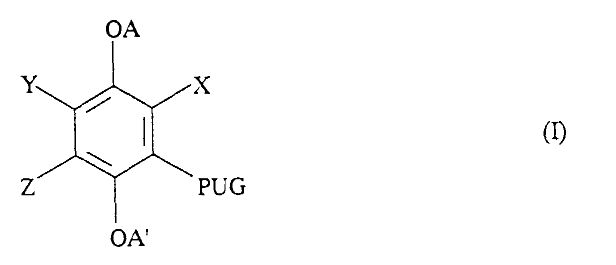
dans laquelle A et A' représentent chacun un atome d'hydrogène ou un groupe qui peut être hydrolysé par un alcali ;
X, Y et Z représentent chacun un atome d'hydrogène, un atome d'halogène, un groupe alkyle substitué ou non substitué, un groupe alcoxy substitué ou non substitué, un groupe aryle substitué ou non substitué ou un groupe alkylthio, X peut représenter en outre un groupe arracheur d'électrons choisi parmi 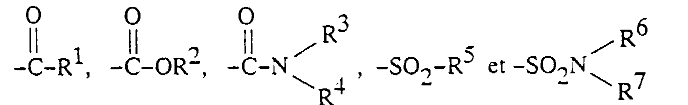
où R¹ à R⁷ représcntent chacun un groupe alkyle substitué ou non substitué, un groupe aryle substitué ou non substitué ou un groupe aralkyle substitué ou non substitué ;
ou bien Y et Z peuvent former un cycle à 5 ou 6 chaînons par l'intermédiaire d'une chaîne méthylène ;
PUG est un groupe libéré par réaction avec un halogénure d'argent exposé à la lumière et/ou le produit oxydé de l'agent de développement et est lié au noyau benzénique par un atome de soufre ou un atome d'azote ;
où ledit PUG est choisi dans le groupe comprenant les formules (P-1) et (P-2) : 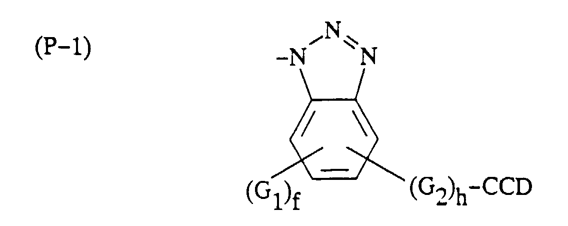 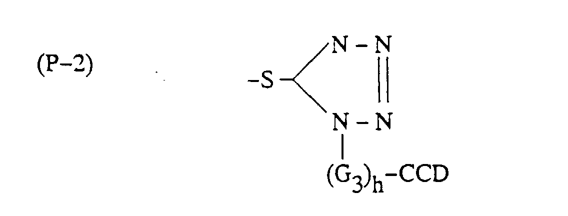
dans lesquelles G₁ est un atome d'hydrogène, un atome d'halogène, un groupe alkyle, un groupe acylamino, un groupe alcoxy, un groupe sulfonamido, un groupe aryle, un groupe alkylthio, un groupe alkylamino, un groupe anilino, un groupe amino, un groupe alcoxycarbonyle, un groupe acyloxy, un groupe nitro, un groupe cyano, un groupe sulfonyle, un groupe aryloxy, un groupe hydroxy, un groupe thioamido, un groupe carbamoyle, un groupe sulfamoyle, un groupe carboxyle, un groupe uréido ou un groupe aryloxycarbonyle ;
G₂ est l'un quelconque des substituants cités pour G₁ qui peut former un groupe divalent ;
G₃ est un groupe alkylène substitué ou non substitué ou un groupe arylène substitué ou non substitué et peut être interrompu par une liaison éther, ester, thioéther, amido, uréido, imido, sulfone ou sulfonamido ou un groupe carbonyle, lesdits groupes de liaison et lesdits groupes alkylène et arylène pouvant être liés ensemble pour former un groupe divalent ;
f est un nombre entier de 1 ou 2 ;
h est 0 ou 1, et
CCD est
(D-1) -COOR⁸
où R⁸ est un groupe alkyle substitué ou non substitué, un groupe aryle substitué ou non substitué ou un groupe aralkyle substitué ou non substitué.

2. Matériau photographique à l'halogénure d'argent selon la revendication 1, dans lequel ledit composé est employé en une quantité de 10⁻⁶ à 1 mol/mol d'argent.

3. Matériau photographique à l'halogénure d'argent selon la revendication 1, dans lequel ledit composé est employé en une quantité de 10⁻⁵ à 10⁻¹ mol/mol d'argent.
